(19) Europäisches Patentamt
European Patent Office
Office européen des brevets

(11) Veröffentlichungsnummer: **0 110 224**
**A2**

# (12) EUROPÄISCHE PATENTANMELDUNG

(21) Anmeldenummer: **83111352.7**

(22) Anmeldetag: **14.11.83**

(51) Int. Cl.³: **C 07 C 103/52**, A 61 K 37/02

(30) Priorität: **24.11.82 DE 3243370**

(43) Veröffentlichungstag der Anmeldung: **13.06.84**
**Patentblatt 84/24**

(84) Benannte Vertragsstaaten: **AT BE CH DE FR GB IT LI NL SE**

(71) Anmelder: **BASF Aktiengesellschaft,**
**Carl-Bosch-Strasse 38, D-6700 Ludwigshafen (DE)**

(72) Erfinder: **Kubinyi, Hugo, Dr., Donnersbergstrasse 9,**
**D-6714 Weisenheim/Sand (DE)**
Erfinder: **Traut, Martin, Dr., Muehltalstrasse 125,**
**D-6900 Heidelberg (DE)**
Erfinder: **Gries, Josef, Dr., Roemerweg 43,**
**D-6706 Wachenheim (DE)**

## (54) Benzoylthioverbindungen, ihre Herstellung und Verwendung als Arzneimittel.

(57) Die Erfindung betrifft Benzoylthioverbindungen der Formel I

$$
\begin{array}{l}
\qquad\qquad\qquad\quad C_6H_5-CO-X \\
\qquad\qquad\qquad\qquad\qquad | \\
\qquad\qquad\qquad\qquad\quad (CH_2)_q \\
\qquad\qquad\qquad\qquad\qquad | \\
C_6H_5-CO-S-(CH_2)_n-CH-CO-NR^1-CHR^2-CO-(NR^3-CHR^4-CO)_m-OH
\end{array} \qquad I,
$$

worin

$R^1$ und $R^3$ gleich oder verschieden sind und Wasserstoffatome oder $C_{1-4}$-Alkylgruppen darstellen,

$R^2$ und $R^4$ gleich oder verschieden sind und Wasserstoff, einen gegebenenfalls durch eine Hydroxy-, Mercapto-, $C_{1-4}$-Alkylthio-, Carbonsäureester-, Carbonamido- oder Acylaminogruppe substituierten $C_{1-6}$-Alkylrest oder einen gegebenenfalls im Arylrest durch Hydroxy-, $C_{1-4}$-Alkoxy-, Arylalkoxygruppen oder Halogenatome substituierten Aryl-, Heteroaryl-, Arylalkylen- oder Heteroarylalkylenrest bedeuten, wobei $R^1$ und $R^2$ und/oder $R^3$ und $R^4$ zusammen auch die Reste $-(CH_2)_p-$ (p = 2, 3, 4), $-CH_2-S-CH_2-$ oder $-CH_2-C_6H_4-CH_2-$(ortho) sein können,

X eine NH-Gruppe oder ein Schwefelatom ist,

m und n gleich oder verschieden sind und die Zahlen 0 oder 1 bedeuten und

q – falls X = S – die Zahl 0 oder 1 oder – falls X = NH – die Zahl 0 darstellt,

sowie deren Salze mit physiologisch verträglichen Basen, deren Herstellung sowie diese enthaltende Arzneimittel und deren Verwendung bei der Bekämpfung von Krankheiten.

EP 0 110 224 A2

# Benzoylthioverbindungen, ihre Herstellung und Verwendung als Arzneimittel

Die Erfindung betrifft neue Benzoylthioverbindungen, deren Herstellung sowie diese enthaltende Arzneimittel und deren Verwendung bei der Bekämpfung von Krankheiten.

Für die Blutdruckregulation höherer Organismen spielt das Renin--Angiotensin-System eine wichtige Rolle. Es ist bekannt, daß Inhibitoren des Angiotensin converting-Enzyms (ACE) blutdrucksenkend wirken und zur Therapie des Hochdrucks beim Menschen eingesetzt werden können. Solche therapeutisch verwendbaren Inhibitoren enthalten Thiolgruppen, Carboxylgruppen oder Phosphamidgruppen, die mit dem funktionellen Zn-Atom des ACE in Wechselwirkung treten (M.A. Ondetti und D.W. Cushman, J. Med. Chem. 24, 355 (1981) und dort zitierte Literatur.) Verbindungen mit blockierter Thiol- bzw. Carboxylgruppe sind - zumindest in vitro - nur sehr schwach wirksam bis unwirksam (D.W. Cushman et al., Biochemistry 16, 5584 (1977), z.B. Verbindung 37; A.A. Patchett et al., Nature 288, 280 (1980), z.B. Verbindung 10). Es wurde nun überraschenderweise gefunden, daß bestimmte Verbindungen mit blockierter Thiolfunktion sowohl in vitro als auch in vivo hochwirksame ACE-Inhibitoren sind.

Die Erfindung betrifft Benzoylthioverbindungen der Formel I

$$
\begin{array}{l}
\qquad\qquad\qquad C_6H_5\text{-}CO\text{-}X \\
\qquad\qquad\qquad\qquad\quad | \\
\qquad\qquad\qquad\qquad (CH_2)_q \\
\qquad\qquad\qquad\qquad\quad | \\
C_6H_5\text{-}CO\text{-}S\text{-}(CH_2)_n\text{-}CH\text{-}CO\text{-}NR^1\text{-}CHR^2\text{-}CO\text{-}(NR^3\text{-}CHR^4\text{-}CO)_m\text{-}OH
\end{array}
\qquad I,
$$

worin

$R^1$ und $R^3$ gleich oder verschieden sind und Wasserstoffatome oder $C_{1-4}$--Alkylgruppen darstellen,

$R^2$ und $R^4$ gleich oder verschieden sind und Wasserstoff, einen gegebenenfalls durch eine Hydroxy-, Mercapto-, $C_{1-4}$-Alkylthio-, Carbonsäureester-, Carbonamido- oder Acylaminogruppe substituierten $C_{1-6}$-Alkylrest oder einen gegebenenfalls im Arylrest durch Hydroxy-, $C_{1-4}$--Alkoxy-, Arylalkoxygruppen oder Halogenatome substituierten Aryl-, Heteroaryl-, Arylalkylen- oder Heteroarylalkylenrest bedeuten, wobei $R^1$ und $R^2$ und/ oder $R^3$ und $R^4$ zusammen auch die Reste $-(CH_2)_p-$ ($p$ = 2, 3, 4), $-CH_2-S-CH_2-$ oder $-CH_2-C_6H_4-CH_2-$(ortho) sein können,

X eine NH-Gruppe oder ein Schwefelatom ist,

WK/ro

m und n gleich oder verschieden sind und die Zahlen 0 oder 1 bedeuten, und

q – falls X = S – die Zahlen 0 oder 1 oder – falls X = NH – die Zahl 0 darstellt,

sowie deren Salze mit physiologisch verträglichen Basen.

Als Reste sind für $R^1$ besonders Wasserstoff, aber auch Methyl und Ethyl zu nennen.

Als Reste für $R^2$ kommen besonders Wasserstoff, Methyl, Ethyl, Propyl, Isopropyl, n-Butyl, Isobutyl und sec.-Butyl in Betracht. Als Substituenten dieser Reste seien speziell der Methylthio-, Ethylthio-, Methoxycarbonyl-, Ethoxycarbonyl-, Benzyloxycarbonyl-, Formamido-, Acetamido-, Benzamido- und Benzyloxycarbamido-Rest genannt. Als ringhaltige Substituenten sind besonders der Phenyl-, Naphthyl-, Thienyl-, Pyrrolyl-, Benzyl-, Naphthylmethylen-, Indolylmethylen-, Pyrrolylmethylen-, Imidazolylmethylen- und Thienylmethylenrest zu nennen. $R^3$ ist besonders ein Wasserstoffatom und $R^4$ ein Wasserstoffatom, ein $C_{1-4}$-Alkyl-, Benzyl- oder Indol-3-yl-methylenrest. $R^1$ und $R^2$ und/oder $R^3$ und $R^4$ zusammen sind auch bevorzugt die Gruppen $-(CH_2)_3-$ oder $-CH_2-S-CH_2-$.

Ganz besonders wirksam sind die Verbindungen, in denen m = 0, $R^1$ ein Wasserstoffatom und $R^2$ den Benzyl-, Naphthylmethylen- oder Indol-3-yl-methylen-Rest bzw. $R^1$ und $R^2$ zusammen den $-(CH_2)_3-$ oder $-CH_2-S-CH_2-$Rest bedeuten, sowie diejenigen, in denen m = 1, $R^1$ ein Wasserstoffatom, $R^2$ ein $C_{1-4}$-Alkylrest und $R^3$ und $R^4$ zusammen die Reste $-(CH_2)_3-$ oder $-CH_2-S-CH_2-$ bedeuten.

X ist vorzugsweise ein Schwefelatom und n ist vorzugsweise 1.

Die neuen Verbindungen werden hergestellt, indem man

a) eine Säure der Formel II

$$\begin{array}{l} \qquad\quad C_6H_5-CO-X \\ \qquad\qquad\qquad\quad | \\ \qquad\qquad\qquad (CH_2)_q \\ \qquad\qquad\qquad\quad | \\ C_6H_5-CO-S-(CH_2)_n-CH-COOH \end{array} \qquad II,$$

worin X, n und q die angegebene Bedeutung besitzen, mit einer Verbindung der Formel III

$R^1NH-CHR^2-CO-(NR^3-CHR^4-CO)_m-OH$ III,

worin $R^1$, $R^2$, $R^3$, $R^4$ und m die angegebene Bedeutung besitzen kondensiert oder

b) - falls X ein S-Atom ist - eine Verbindung der Formel

$$\begin{array}{l} \quad\quad\quad Y-(CH_2)_q \\ Y-(CH_2)_n-CH-CO-NR^1-CHR^2-CO-(NR^3-CHR^4-CO)_m-OH \end{array} \quad (IV),$$

worin $R^1$, $R^2$, $R^3$, $R^4$, m, n und q die angegebene Bedeutung besitzen und Y ein Halogenatom ist mit Thiobenzoesäure umsetzt

und die so erhaltenen Verbindungen gegebenenfalls in ihre Salze mit physiologisch verträglichen Basen überführt.

Die Säuren (II) werden zur Umsetzung gemäß Verfahren a) in ihre carboxylaktivierten Derivate übergeführt. Als solche eignen sich besonders die Säurechloride, aber auch Säurebromide, Arylester, wie z.B. p-Nitrophenylester, asymmetrische Anhydride, wie z.B. Alkyl- oder Aralkylkohlensäureanhydride, N-Acylazole, wie z.B. Imidazol-N-carbonsäureanhydride, O-Acyl-N,N-diacyl-hydroxylamine, wie z.B. N-Hydroxysuccinimidester, und andere in der Peptidchemie verwendete carboxylaktivierte Derivate (Houben-Weyl, Methoden der organischen Chemie, Bd. XV, Teil 2, S. 2 bis 364). Zur Herstellung der Säurechloride setzt man vorteilhafterweise die Säuren II in einem wasserfreien aprotischen Lösungsmittel, wie z.B. Benzol, Toluol oder Dioxan mit überschüssigem Oxalsäuredichlorid um. Man kann jedoch statt Oxalsäuredichlorid auch Thionylchlorid, Phosphortrichlorid, Phosphorpentachlorid oder andere Chlorierungsreagentien verwenden (s. z.B. Fieser und Fieser, Reagents for Organic Synthesis, Bd. I-X).

Die carboxylaktivierten Säurederivate werden direkt mit den Verbindungen III umgesetzt. Diese können in der D,L-Form, D-Form oder bevorzugt in der natürlich vorkommenden L-Form verwendet werden.

Die Reaktion wird in einem polaren aprotischen organischen Lösungsmittel, wie z.B. Dioxan, Tetrahydrofuran, Ethylenglykoldimethylether, Diethylenglykoldimethylether, Acetonitril, Dimethylformid oder Dimethylsulfoxid bei erhöhter Temperatur, d.h. zwischen etwa 70 und 120°C, vorteilhafterweise bei der Siedetemperatur des organischen Lösungsmittels, durchgeführt. Die erhaltenen Reaktionsprodukte können nach üblicher Aufarbeitung entweder direkt oder nach säulenchromatographischer Reinigung, bevorzugt

an desaktiviertem Kieselgel, kristallisiert werden. Dabei können Diastereomerengemische der Formel I, die sich aus der Umsetzung von racemischen Säuren der Formel II mit racemischen bzw. optisch aktiven Aminosäuren oder Dipeptiden der Formel III ergeben, durch säulenchromatographische Trennung oder durch fraktionierte Kristallisation in die Einzelkomponenten getrennt werden.

Zur Herstellung von Verbindungen der Formel I kann man auch von den freien Säuren der Formel II ausgehen und sie mit einer durch eine säurelabile Schutzgruppe carboxylgeschützten Aminosäure oder einem Dipeptid der Formel III umsetzen. Als säurelabile Schutzgruppe eignet sich insbesondere der tert.-Butylrest, es können aber auch andere aus der Peptidchemie bekannte Schutzgruppen, wie z.B. der 4-Methoxybenzyl-, der Diphenylmethyl- oder der Triphenylmethylrest verwendet werden (s. Houben-Weyl, Bd. XV, Teil 1, S. 315 bis 405). Die Aminosäuren bzw. Dipeptide können auch hier in der D,L-Form, der D-Form oder bevorzugt in der natürlich vorkommenden L-Form eingesetzt werden. Die Umsetzung erfolgt in einem geeigneten organischen Lösungsmittel in Gegenwart eines in der Peptidchemie gebräuchlichen Kopplungs-Reagens, z.B. Dicyclohexylcarbodiimid oder 1-Ethyl-3-(3-dimethylaminopropyl)-carbodiimid-hydrochlorid (s. Houben-Weyl, Bd. XV, Teil 2, S. 103 bis 117); bei Verwendung von 1-Ethyl-3-(3-dimethylaminopropyl)-carbodiimid-hydrochlorid kann die Umsetzung auch in organisch-wäßrigem Medium durchgeführt werden.

Die erhaltenen eine Schutzgruppe tragenden Reaktionsprodukte werden als Rohprodukte bzw. nach Reinigung durch Kristallisation oder Säulenchromatographie in die Verbindungen der Formel I gespalten. Als Säuren, mit denen die Spaltung durchgeführt werden kann, eignen sich insbesondere HCl/Dioxan-Gemische, aber auch Trifluoressigsäure oder HBr/Eisessig-Gemische (s. Houben-Weyl, Bd. XV, Teil I, S. 315 bis 405).

Auch hier können Diasteromeren-Gemische durch säulenchromatographische Trennung oder durch fraktionierte Kristallisation in die Einzelkomponenten getrennt werden. Die Trennung kann sowohl auf der Stufe der carboxylgeschützten Derivate als auch auf der Stufe der Endprodukte erfolgen.

Eine bevorzugte Ausführungsform der Erfindung besteht darin, daß man die verwendeten Säuren der allgemeinen Formel II, sofern diese ein asymmetrisches C-Atom besitzen, in Form der optisch reinen Antipoden einsetzt. Dazu kann entweder ein Racemat der Säure II mit Hilfe einer optisch aktiven Base, z.B. (+)- oder (-)-Ephedrin, in die Antipoden gespalten werden oder aber ein Antipode der Säure II durch Benzoylierung einer optisch aktiven Vorstufe dargestellt werden.

Die Umsetzung der optischen Antipoden der Säuren II erfolgt zweckmäßigerweise mit carboxylgeschützten Aminosäuren bzw. Dipeptiden, da bei zu starker Aktivierung der Carboxylgruppe der Säure II, z.B. über die Säurechloride, die Gefahr der teilweisen bzw. vollständigen Racemisierung der Säure II besteht. Bei entsprechend schonender Aktivierung der Säure-Carboxylgruppe, z.B. über die N-Hydroxysuccinimidester oder mit Carbodiimiden erfolgt dagegen keine nennenswerte Racemisierung.

Das Verfahren b) wird vorzugsweise mit den Verbindungen der Formel IV durchgeführt, in denen Y Brom, Chlor oder Iod bedeutet (Ber. 37, 2486 (1904), Fermentf. 10, 213 (1928) = C. 1929 I, 2319, J. Chem. Soc. Perkin I, 1972, 2121). Diese Verbindungen werden in einem aprotischen Lösungsmittel, vorzugsweise Toluol, Dioxan, Tetrahydrofuran, Ethylenglycoldimethylether, Acetonitril, Dimethylformamid oder Dimethylsulfoxid, in Gegenwart einer anorganischen Base, vorzugsweise wasserfreiem Kaliumcarbonat, bei Temperaturen zwischen 0 und 40°C mit Thiobenzoesäure umgesetzt.

Die als Ausgangsmaterial verwendeten Säuren der Formel II, in der X gleich -NH-, n gleich 0 oder 1 und q = 0 ist, werden durch Umsetzung von Oxosäuren der allgemeinen Formel V

$$H-(CH_2)_r-\overset{\overset{O}{\parallel}}{C}-COOH \qquad (V),$$

in der r gleich 0 oder 1 ist, mit Benzamid und Thiobenzoesäure dargestellt. Dazu werden die Reaktionspartner in etwa äquimolaren Mengen in einem aprotischen organischen Lösungsmittel, z.B. Benzol, Toluol oder Dioxan, bei erhöhter Temperatur, bevorzugt bei der Siedetemperatur des Lösungsmittels, gegebenenfalls auch unter laufender Abscheidung des während der Umsetzung entstehenden Reaktionswassers, umgesetzt. Während bei Verwendung der Säure V mit r = 0 erwartungsgemäß ein N,S-Acetal entsteht, bildet sich bei Verwendung der Säure V mit r = 1 intermediär ein N,S-Ketal, das nach Abspaltung und Michael-Addition von Thiobenzoesäure in die gewünschte Säure II mit X gleich -NH-, n gleich 1 und q = 0 übergeht.

Die Ausgangsstoffe der Formel II, in denen X ein Schwefelatom ist, sind beispielsweise durch Umsetzen von Verbindungen der Formel VI

$$\begin{array}{c} Y \\ | \\ (CH_2)_q \\ | \\ Y-(CH_2)_n-CH-COOH \end{array} \qquad VI,$$

in der n, q und Y die genannte Bedeutung haben, mit Thiobenzoesäure zugänglich. Die Reaktion läßt sich in Gegenwart von Kaliumcarbonat in Acetonitril oder Dioxan/Wasser bei 0 bis 40°C durchführen.

Die erfindungsgemäßen Verbindungen können mit Basen in üblicher Weise in physiologisch verträgliche Salze überführt werden. Geeignete Salze sind beispielsweise Ammoniumsalze, Alkalimetallsalze, insbesondere des Natriums, Kaliums und Lithiums, Erdalkalimetallsalze, insbesondere des Calciums oder Magnesiums, sowie Salze mit geeigneten organischen Basen, wie mit niederen Alkylaminen, z.B. Methylamin oder Ethylamin, mit substituierten niederen Alkylaminen, insbesondere hydroxysubstituierten Alkylaminen, wie Diethanolamin, Triethanolamin, Tris-(hydroxymethyl)-aminomethan oder Diethylaminoethanol oder Morpholin.

Die erfindungsgemäßen Verbindungen und ihre physiologisch verträglichen Salze sind als Pharmaka mit Angiotensin Converting Enzyme hemmender Wirkung zur Behandlung der Hypertonie geeignet.

Zur Untersuchung der pharmakodynamischen Wirkung wurde folgende Methode verwendet (Hemmung der Angiotensin Converting Enzyme ex vivo bei Ratten):

Vor und 2 h nach Substanzgabe (p.o.) wurde den Tieren Blut abgenommen und das Plasma mit $H_2O$ 1:6 verdünnt. Das verdünnte Plasma (100 µl) wurde mit 50 µl $^{14}C$-Hippuryl-histidyl-leucin-Lösung (K-Phosphat 260 mM; NaCl 938 mM, pH = 8,3) 1 h bei 37°C inkubiert. Die Reaktion wurde durch Zugabe von 2 ml 0,1 N HCl gestoppt, das Reaktionsprodukt $^{14}C$-Hippursäure mit Toluol-Szintillator (PPO + POPOP + Toluol + 21 % Isoamylalkohol) extrahiert und im Flüssigkeits-Szintillationszähler bestimmt. Nach Abzug des Blindwertes (Inkubationszeit = 0 min) wurde die prozentuale Hemmung aus den enzymatischen Aktivitäten im Plasma vor und nach Substanzgabe ermittelt. Die ED 50 (Dosis mit 50 % Hemmung) wird aus den einzelnen Hemmwerten durch lineare Regression nach logit-log-Transformation berechnet.

Aus der Tabelle geht die überlegene Wirksamkeit der erfindungsgemäßen Verbindungen hervor. So führen die Substanzen der Beispiele 68 und 70 in 5 mal kleineren Dosen als Captopril zu einer Hemmung der Angiotensin Converting Enzyme.

Tabelle
Angiotensin Converting Enzyme hemmende Wirkung

| Substanz des Beispiels | ED 50 % [1] mg/kg | R.W. |
|---|---|---|
| 68 | 0,2 | 5,0 |
| 70 | 0,2 | 5,0 |
| Captopril | 1,00 | 1,00 |

[1] Dosis, welche eine Hemmung um 50 % bewirkt.

Die erfindungsgemäßen Verbindungen können in üblicher Weise oral oder parenteral (subkutan, intravenös, intramuskulär, intraperitoneal) verabfolgt werden. Die Applikation kann auch mit Dämpfen oder Sprays durch den Nasen-Rachenraum erfolgen.

Die Dosierung hängt vom Alter, Zustand und Gewicht des Patienten sowie von der Applikationsart ab. In der Regel beträgt die tägliche Wirkstoffdosis bei oraler Gabe zwischen etwa 0,1 und 10 mg/kg Körpergewicht. Im Normalfall werden mit täglichen Dosen von 0,2 bis 2,0 mg/kg oral zufriedenstellende Ergebnisse erzielt.

Die neuen Verbindungen können in den gebräuchlichen galenischen Applikationsformen fest oder flüssig angewendet werden, z.B. als Tabletten, Filmtabletten, Kapseln, Pulver, Granulate, Dragees, Suppositorien, Lösungen, Salben, Cremes oder Sprays. Diese werden in üblicher Weise hergestellt. Die Wirkstoffe können dabei mit den üblichen galenischen Hilfsmitteln wie Tablettenbindern, Füllstoffen, Konservierungsmitteln, Tablettensprengmitteln, Fließreguliermitteln, Weichmachern, Netzmitteln, Dispergiermitteln, Emulgatoren, Lösungsmitteln, Retardierungsmitteln, Antioxidantien und/oder Treibgasen verarbeitet werden (vgl. H. Sucker et al: Pharmazeutische Technologie, Thieme-Verlag, Stuttgart, 1978). Die so erhaltenen Applikationsformen enthalten den Wirkstoff normalerweise in einer Menge von 0,1 bis 99 Gewichtsprozent.

Die folgenden Beispiele sollen die Erfindung erläutern.

Alle Schmelzpunkte sind unkorrigiert. Angaben zur Dünnschichtchromatographie beziehen sich auf die Systeme
I = Essigsäureethylester/Eisessig = 20/1
II = Cyclohexan/Essigsäureethylester/Eisessig = 10/10/1.

Die Dünnschichtchromatographie (DC) wurde an Kieselgel 60 $F_{254}$-Platten der Firma Merck, Darmstadt unter Kammersättigung durchgeführt. Die Laufstrecke betrug ca. 13 cm. Sofern in den Beispielen die Aminosäuren ohne D bzw. DL bezeichnet werden, handelt es sich um die L-Verbindungen.

Beispiel 1

N-[N-Benzoyl-α-(benzoylthio)-glycyl]-glycin

a) Herstellung des Ausgangsmaterials

9,2 g (0,1 Mol) Glyoxylsäuremonohydrat wurden zusammen mit 12,1 g (0,1 Mol) Benzamid und 15,2 g (0,11 Mol) Thiobenzoesäure in 200 ml trockenem Toluol 3 h unter Rückfluß gekocht. Das entstehende Reaktionswasser wurde laufend über einen Wasserabscheider ausgekreist. Nach Abkühlen der Reaktionslösung, Filtration und Waschen mit Toluol erhielt man 25,4 g (81 %) N-Benzoyl-α-(benzoylthio)-glycin, Fp. 165 bis 168°C, $R_f$ = 0,27 in System I.

b) Herstellung des Endprodukts

16,8 g (0,1 Mol) Glycin-tert.-butylester-hydrochlorid wurden in 100 ml trockenem Acetonitril suspendiert, mit 10,1 g (0,1 Mol) Triethylamin versetzt und kurz bei Raumtemperatur gerührt. Der Ansatz wurde mit einer Lösung von 31,5 g (0,1 Mol) N-Benzoyl-α-(benzoylthio)-glycin in 400 ml trockenem Acetonitril und 20,6 g (0,1 Mol) Dicyclohexylcarbodiimid versetzt und 3 h bei Raumtemperatur gerührt. Der ausgefallene Dicyclohexylharnstoff wurde abfiltriert, das Filtrat unter vermindertem Druck eingedampft und der Rückstand mit Essigsäureethylester aufgenommen. Nach nochmaligem Filtrieren und Eindampfen des Filtrats wurde der Rückstand an desaktiviertem Kieselgel mit Cyclohexan/Essigsäureethylester = 1/1 säulenchromatographiert. Man erhielt nach Kristallisation aus Essigsäureethylester 27,8 g N-[N-Benzoyl-α-(benzoylthio)-glycyl]-glycin-tert.-butylester, Fp. 157 bis 160°C, $R_f$ = 0,50 in Cyclohexan/ Essigsäureethylester = 1/1. Das Produkt wurde in 100 ml trockenem Dioxan gelöst und mit 100 ml 6N HCl/Dioxan 3 h bei 20°C stehen gelassen. Der Ansatz wurde anschließend mit 500 ml Essigsäureethylester versetzt und die organische Phase dreimal mit je 500 ml Wasser ausgeschüttelt. Nach Trocknen der organischen Phase mit wasserfreiem Natriumsulfat und Eindampfen unter vermindertem Druck wurde der Rückstand aus Diethylether kristallisiert. Man erhielt 22,8 g (61 %) N-[N-Benzoyl-α-(benzoylthio)-glycyl]-glycin, Fp. 194 bis 197°C, $R_f$ = 0,31 in System I.

Analog wurde hergestellt:

2. N-[N-Benzoyl-α-(benzoylthio)-glycyl]-alanin als Diastereomerengemisch, Fp. 162 bis 164°C (Diethylether), $R_f$ = 0,47 und 0,40 in System I.

Beispiel 3

N-[N-Benzoyl-α-(benzoylthio)-glycyl]-prolin

Man verfuhr wie in Beispiel 1b (ohne Zusatz von Triethylamin) und verwendete Prolin-tert.-butylester statt Glycin-tert.-butylester-hydrochlorid. So wurde N-[N-Benzoyl-α-(benzoylthio)-glycyl]-prolin als öliges Diastereomerengemisch erhalten. Kristallisation aus Essigsäureethylester lieferte das unpolare Isomere, Fp. 202 bis 205°C, $R_f$ = 0,34 in System I, $[\alpha]_D^{20}$= +25° (c = 1 in Chloroform). Kristallisation der Mutterlaugen aus Diethylether/ Petrolether lieferte das polare Isomere, Fp. 182 bis 185°C, $R_f$ = 0,30 in System I, $[\alpha]_D^{20}$= -87° (c = 1 in Chloroform).

Beispiel 4

N-[N-Benzoyl-α-(benzoylthio)-glycyl]-tryptophan

Man verfuhr wie in Beispiel 1b (ohne Zusatz von Triethylamin) und verwendete Tryptophan-tert.-butylester statt Glycin-tert.-butylester-hydrochlorid. Bei der sauren Spaltung des tert.-Butylesters wurde dem Ansatz zur Vermeidung von Nebenreaktionen des Indolringes Ethandithiol zugesetzt. Nach Kristallisation aus Diethylether erhielt man N-[N-Benzoyl-α--(benzoylthio)-glycyl]-tryptophan als Diastereomerengemisch, Fp. 188 bis 191°C. Säulenchromatographische Trennung des Gemisches an desaktiviertem Kieselgel mit Cyclohexan/Essigsäureethylester = 3/2 lieferte das unpolare Isomere, Fp. 180 bis 183°C, $R_f$ = 0,51 in System I, $[\alpha]_D^{20}$= +18° (c = 1 in Chloroform/ Methanol = 9/1) und das polare Isomere, Fp. 200 bis 203°C, $R_f$ = 0,46 in System I, $[\alpha]_D^{20}$= 0° (c = 1 in Chloroform/Methanol = 9/1).

Beispiel 5

N-[N-Benzoyl-α-(benzoylthio)-glycyl]-alanyl-prolin

12,1 g (50 mMol) Alanyl-prolin-tert.-butylester (hergestellt durch Kopplung von Benzyloxycarbonylalanin und Prolin-tert.-butylester mit Dicyclohexylcarbodiimid und nachfolgende hydrogenolytische Abspaltung der Schutzgruppe) wurden in 150 ml trockenem Acetonitril gelöst, mit einer Lösung von 15,8 g (50 mMol) N-Benzoyl-α-(benzoylthio)-glycin in 150 ml trockenem Tetrahydrofuran und 10,3 g (50 mMol) Dicyclohexylcarbodiimid versetzt und über Nacht bei Raumtemperatur gerührt. Der ausgefallene Dicyclohexylharnstoff wurde abgesaugt und das Filtrat unter vermindertem Druck eingedampft. Der Rückstand wurde an desaktiviertem Kieselgel mit Cyclohexan/Essigsäureethylester = 1/1 säulenchromatographiert. Man erhielt 20,2 g (75 %) N-[N-Benzoyl-α-(benzoylthio)-glycyl]-alanyl-prolin-tert.-butylester als Diastereomerengemisch. Kristallisation aus Cyclohexan/Essigsäureethylester lieferte 7,5 g eines niedrigschmelzenden Isomeren, Fp. 97 bis 100°C; aus der Mutterlauge erhielt man nach zweimaliger Kristallisation aus Cyclohexan/Essigsäureethylester 7,8 g eines hochschmelzenden Isomeren, Fp. 147 bis 150°C. Getrennte Spaltung der beiden Isomeren mit 6N HCl/Dioxan analog Beispiel 1b lieferte N-[N-Benzoyl-α-(benzoylthio)-glycyl]-alanyl-prolin als Diastereomeres I, Kristalle aus Essigsäureethylester, Fp. 185 bis 188°C, $R_f$ = 0,18 in System I, $[\alpha]_D^{20}$= +29° (c = 1 in Chloroform), und als Diastereomeres II, Kristalle aus Essigsäureethylester, Fp. 203 bis 206°C, $R_f$ = 0,18 in System I, $[\alpha]_D^{20}$= -167° (c = 1 in Chloroform).

Beispiel 6

N-[2,2-Bis-(benzoylthio)-acetyl]-glycin

Zu einer gut gerührten Suspension von 55,3 g (0,4 Mol) wasserfreiem Kaliumcarbonat in 100 ml trockenem Acetonitril tropfte man bei 0 bis 5°C eine Lösung von 27,5 g (0,1 Mol) N-(2,2-Dibromacetyl)-glycin und 30,4 g (0,22 Mol) Thiobenzoesäure in 100 ml trockenem Acetonitril. Man ließ noch 1 h bei 15 bis 20°C nachrühren, trug den Ansatz anschließend in 1 l Eiswasser ein und extrahierte zweimal mit je 400 ml Diethylether. Die vereinigten Etherphasen wurden noch einmal mit 500 ml 10 %iger wäßriger Kaliumcarbonatlösung extrahiert und die vereinigten wäßrigen Phasen unter Kühlung mit 2N wäßriger $H_2SO_4$ auf pH 3 gebracht. Man extrahierte zweimal mit je 400 ml Essigsäureethylester, trocknete die organische Phase mit wasserfreiem Natriumsulfat und dampfte ein. Nach Säulenchromatographie an desaktiviertem Kieselgel mit dem System Cyclohexan/ Essigsäureethylester = 2/1 erhielt man 24,5 g (63 %) N-[2,2-Bis-(benzoylthio)-acetyl]-glycin, Kristalle aus Diisopropylether, Fp. 141 bis 143°C, $R_f$ = 0,50 in System I.

Beispiel 7

N-[2,2-Bis-(benzoylthio)-acetyl]-alanin

Herstellung des Ausgangsmaterials

a) Zu einer gut gerührten Suspension von 110,6 g (0,8 Mol) wasserfreiem Kaliumcarbonat in 150 ml trockenem Acetonitril tropfte man bei 0 bis 5°C eine Lösung von 43,6 g (0,2 Mol) Dibromessigsäure und 60,8 g

(0,44 Mol) Thiobenzoesäure in 100 ml trockenem Acetonitril. Man ließ noch 45 min bei 15 bis 20°C nachrühren, trug den Ansatz anschließend in 1 l Eiswasser ein und extrahierte zweimal mit je 400 ml Diethylether. Die vereinigten Etherphasen wurden noch einmal mit 500 ml 10 %iger wäßriger Kaliumcarbonatlösung extrahiert und die vereinigten wäßrigen Phasen unter Kühlung mit 2N wäßriger $H_2SO_4$ auf pH 3 gebracht. Man extrahierte zweimal mit je 400 ml Essigsäureethylester, trocknete die organischen Phasen mit wasserfreiem Natriumsulfat und dampfte ein. Das zurückbleibende Öl wurde aus Benzol kristallisiert. Nach Absaugen und Waschen mit Benzol/Petrolether erhielt man 48,4 g (59 %) 2,2-Bis-(benzoylthio)-essigsäure als Komplex mit 1 Mol Kristallbenzol, Fp. 82 bis 83°C; $R_f$ = 0,52 in System I.

b) 16,4 g (40 mMol) 2,2-Bis-(benzoylthio)-essigsäure wurden in 150 ml Toluol suspendiert und unter Kühlung mit 25,4 g (0,2 Mol) Oxalsäuredichlorid versetzt. Man erwärmte 2 h auf 50°C, dampfte anschließend unter vermindertem Druck ein und entfernte überschüssiges Oxalsäuredichlorid durch mehrmaliges Koevaporieren mit Toluol. Das verbleibende rohe 2,2-Bis-(benzoylthio)-acetylchlorid kann direkt für weitere Umsetzungen verwendet werden.

Herstellung des Endprodukts

14,2 g (40 mMol) gemäß b) erhaltenes Rohprodukt wurde in 100 ml trockenem Dioxan gelöst und zu einer Suspension von 3,6 g (40 mMol) Alanin in 100 ml trockenem Dioxan gegeben. Das Reaktionsgemisch wurde 2 h unter Rückfluß gekocht und anschließend das Lösungsmittel unter vermindertem Druck abgezogen. Das ölige Rohprodukt wurde an desaktiviertem Kieselgel mit Cyclohexan/Essigsäureethylester = 2/1 säulenchromatographiert. Man erhielt 10,3 g (64 %) N-[2,2-Bis-(benzoylthio)-acetyl]-alanin, Fp. 161 bis 162°C (Benzol), $R_f$ = 0,56 in Sytem I.

Analog wurden erhalten:

N-[2,2-Bis-(benzoylthio)-acetyl]-glycin, vgl. Beispiel 6

8. N-[2,2-Bis-(benzoylthio)-acetyl]-leucin, Fp. 104 bis 105°C (Tetrachlorkohlenstoff), $R_f$ = 0,63 in System I.

9. N-[2,2-Bis-(benzoylthio)-acetyl]-methionin, Fp. 121 bis 122°C (Benzol/Cyclohexan), $R_f$ = 0,58 in System I.

10. N-[2,2-Bis-(benzoylthio)-acetyl]-prolin, Fp. 80 bis 87°C (nach Fällung aus Diethylether/Petrolether), $R_f$ = 0,49 in System I.

11. N-[2,2-Bis-(benzoylthio)-acetyl]-phenylalanin, Fp. 161 bis 162°C (Benzol), $R_f$ = 0,59 in System I.

12. N-[2,2-Bis-(benzoylthio)-acetyl]-p-chlorphenylalanin, Fp. 141 bis 142°C (Benzol), $R_f$ = 0,58 in System I.

13. D,L-2-[2,2-Bis-(benzoylthio)-acetamido]-4-phenylbuttersäure, Fp. 157 bis 158°C (Benzol), $R_f$ = 0,60 in System I.

14. N-[2,2-Bis-(benzoylthio)-acetyl]-tryptophan, Fp. ab 80°C (Diethylether/Petrolether), $R_f$ = 0,58 in System I.

15. N-[2,2-Bis-(benzoylthio)-acetyl]-alanyl-prolin, Fp. 199 bis 200°C (Essigsäureethylester), $R_f$ = 0,32 in System I.

<u>Beispiel 16</u>

N-(N,S-Dibenzoyl-D,L-cysteinyl)-glycin

Herstellung von Ausgangsmaterialien

a) 264 g (3,0 Mol) frisch destillierte Brenztraubensäure wurden in 2 l Toluol zusammen mit 242,3 g (2,0 Mol) Benzamid und 304,0 g (2,2 Mol) Thiobenzoesäure 3 h unter Rückfluß gekocht. Das entstehende Reaktionswasser und ein Teil der überschüssigen Brenztraubensäure wurden laufend über einen Wasserabscheider ausgekreist. Der Ansatz wurde auf 70°C abgekühlt und das ausgefallene Produkt filtriert. Nach Waschen mit warmem Toluol und Petrolether erhielt man 368,9 g (56 %, bezogen auf eingesetztes Benzamid) N,S-Dibenzoyl-D,L-cystein, Fp. 187 bis 188°C, $R_f$ = 0,43 in System I.

b) 63,0 g (0,4 Mol) Cystein-hydrochlorid wurden in 400 ml Wasser suspendiert und mit 112,5 g (0,8 Mol) Benzoylchlorid versetzt. Unter Rühren und Kühlung auf 0 bis 5°C wurde eine Lösung von 89,8 g (1,6 Mol) Kaliumhydroxid in 1 l Wasser zugetropft. Nach einstündigem Nachrühren bei Raumtemperatur wurde mit 2N wäßriger HCl stark sauer gestellt, der Niederschlag abgesaugt, mit Wasser bis zur neutralen Reaktion gewaschen und das Reaktionsprodukt noch zweimal aus Methanol/Wasser umkristallisiert. Man erhielt 106,3 g (81 %) N,S-Dibenzoyl-cystein, Fp. 183 bis 185°C, $R_f$ = 0,43 in System I, $[\alpha]_D^{20}$= -85,7° (c = 1 in abs. Ethanol).

Herstellung des Endprodukts

Man verfuhr wie in Beispiel 1b und verwendete N,S-Dibenzoyl-D,L-cystein statt N-Benzoyl- -(benzoylthio)-glycin. Wegen der schlechten Löslichkeit des Dibenzoylcysteins wurden dem Reaktionsansatz weitere 400 ml Acetonitril und 200 ml trockenes Dimethylformamid zugegeben. Nach Aufarbeitung und Abspaltung der tert.-Butylgruppe wie in Beispiel 1b erhielt man N-(N,S-Dibenzoyl-D,L-cysteinyl)-glycin, Fp. 184 bis 187°C (Dioxan/Essigsäureethylester), $R_f$ = 0,30 in System I.

Analog Beispiel 16 wurden erhalten:

17. N-(N,S-Dibenzoyl-cysteinyl)-glycin, Fp. 201 bis 204°C (Dioxan), $R_f$ = 0,30 in System I.

18. N-(N,S-Dibenzoyl-D,L-cysteinyl)-alanin als Diastereomerengemisch, Fp. 160 bis 182°C (Diethylether), $R_f$ = 0,45 und 0,40 in System I.

19. N-(N,S-Dibenzoyl-cysteinyl)-alanin, Fp. 181°C (Diethylether), $R_f$ = 0,44 in System I.

20. N-(N,S-Dibenzoyl-cysteinyl)-D-alanin, Fp. 184 bis 185°C (Essigsäureethylester/Diethylether), $R_f$ = 0,41 in System I.

21. N-(N,S-Dibenzoyl-D,L-cysteinyl)-prolin als Diastereomerengemisch, Fp. 85 bis 88°C (Petrolether), $R_f$ = 0,36 und 0,31 in System I.

22. N-(N,S-Dibenzoyl-cysteinyl)-prolin, Fp. 83 bis 86°C (Petrolether), $R_f$ = 0,36 in System I.

23. N-(N,S-Dibenzoyl-cysteinyl)-tryptophan, Fp. 212 bis 214°C (Diethylether), $R_f$ = 0,19 in System II.

24. N-(N,S-Dibenzoyl-D,L-cysteinyl)-alanyl-prolin als Diastereomerengemisch, Fp. 190 bis 193°C (Diethylether), $R_f$ = 0,15 in System I.

25. N-(N,S-Dibenzoyl-cysteinyl)-alanyl-prolin, Fp. 215 bis 217°C (Wasser), $R_f$ = 0,15 in System I.

Beispiel 26

N-(N,S-Dibenzoyl-D,L-cysteinyl)-tryptophan

Analog Beispiel 16 erhielt man mit N,S-Dibenzoyl-D,L-cystein und Tryptophan-tert.-butylester als Ausgangsmaterialien N-(N,S-Dibenzoyl-D,L-cysteinyl)-tryptophan als Diastereomerengemisch, Fp. 175 bis 178°C (Petrolether), $R_f$ = 0,19 und 0,16 in System II. Umkristallisation aus Diethylether lieferte DC-reines N-(N,S-Dibenzoyl-cysteinyl)-tryptophan. Fp. 210 bis 213°C, $R_f$ = 0,19 in System II. Aus der Mutterlauge erhielt man nach Kristallisation aus Petrolether DC-reines N-(N,S-Dibenzoyl-D-cysteinyl)-tryptophan, Fp. 157 bis 160°C, $R_f$ = 0,16 in System II.

Beispiel 27

N-[2,3-Bis-(benzoylthio)-propionyl]-glycin

Verfahren A

a) Herstellung des Ausgangsmaterials

Zu einer auf 0°C gekühlten, gut gerührten Suspension von 276,4 g (2,0 Mol) wasserfreiem, gepulvertem Kaliumcarbonat in 700 ml Toluol wurden 115,9 g (0,5 Mol) 2,3-Dibrompropionsäure und 138,2 g (1,0 Mol) Thiobenzoesäure gegeben. Man rührte noch kurze Zeit bei 0°C und ließ dann die Temperatur des Reaktionsgemisches langsam ansteigen. Bei etwa 20°C trat spontan exotherme Umsetzung ein; durch externe Kühlung mit Eiswasser wurde die Reaktionstemperatur unter 40°C gehalten. Nach Ende der Reaktion wurde mit 1 l Toluol verdünnt, abdekantiert, der Rückstand noch einmal mit Toluol digeriert und in 1 l Eiswasser eingetragen. Nach Ansäuern mit 2N wäßriger $H_2SO_4$ wurde das Reaktionsprodukt zweimal mit je 500 ml Essigsäureethylester extrahiert. Die organischen Phasen wurden mit wasserfreiem Natriumsulfat getrocknet und anschließend unter vermindertem Druck eingedampft. Nach Kristallisation aus Tetrachlorkohlenstoff/Cyclohexan erhielt man 117,8 g (68 %) 2,3-Bis-(benzoylthio)-propionsäure, Fp. 116 bis 118°C, $R_f$ = 0,41 in System II.

200 g der so erhaltenen racemischen 2,3-Bis-(benzoylthio)-propionsäure wurden in 1,5 l Isopropanol gelöst und mit einer Lösung von 106 g (-)-Ephedrin in 500 ml Isopropanol versetzt. Die resultierenden Kristalle wurden aus Methylenchlorid/Isopropanol umkristallisiert. Man erhielt 135,2 g Salz, Fp. 127 bis 129°C, $[\alpha]_D^{20}$= -28,9° (c = 1 in Chloroform). Nach Freisetzung der Säure durch Ausschütteln zwischen Essigsäureethylester und 10 %iger wäßriger Kaliumhydrogensulfat-Lösung und Kristallisation des Eindampfrückstandes der organischen Phase aus Diethylether/Petrolether erhielt man zuerst ein Gemisch aus Racemat und (R)-Form und

anschließend aus der Mutterlauge 33,7 g (R)-2,3-Bis-(benzoylthio)-propionsäure, Fp. 95 bis 96°C, $[\alpha]_D^{20}$= -99° (c = 1 in Chloroform). Aus der Mutterlauge der Kristallisation des (-)-Ephedrin-Salzes wurde die Säure in Freiheit gesetzt und ein Salz mit (+)-Ephedrin gebildet. Man erhielt 97,8 g Salz, Fp. 127 bis 129°C, $[\alpha]_D^{20}$= +33,9° (c = 1 in Chloroform). Spaltung des Salzes und Kristallisation aus Diethylether/Petrolether lieferte zuerst ein Gemisch aus Racemat und (S)-Form und anschließend aus der Mutterlauge 26,6 g (S)-2,3-Bis-(benzoylthio)-propionsäure, Fp. 95 bis 96°C, $[\alpha]_D^{20}$= +103° (c = 1 in Chloroform). Neben den beiden Antipoden wurden insgesamt 122,4 g racemische 2,3-Bis-(benzoylthio)-propionsäure wiedergewonnen, die erneut für eine Racematspaltung eingesetzt werden können.

Analog Beispiel 7b erhielt man aus der racemischen Säure das Säurechlorid. Diese kann direkt für die weiteren Umsetzungen verwendet werden.

b) Herstellung des Endprodukts

18,4 g (50 mMol) des gemäß a) erhaltenen racemischen Säurechlorids wurden in 100 ml trockenem Dioxan gelöst und zu einer Suspension von 3,8 g (50 mMol) Glycin in 100 ml trockenem Dioxan gegeben. Das Reaktionsgemisch wurde 2 h unter Rückfluß gekocht und anschließend das Lösungsmittel unter vermindertem Druck abgezogen. Kristallisation des Rückstandes aus Essigsäureethylester lieferte 9,3 g (46 %) N-[2,3-Bis-(benzoylthio)-propionyl]-glycin, Fp. 160 bis 163°C, $R_f$ = 0,59 in System I. Aus der Mutterlauge erhielt man nach Säulenchromatographie an desaktiviertem Kieselgel mit dem System Cyclohexan/Essigsäureethylester = 1/1 weiteres DC-reines Produkt, Fp. 162 bis 164°C.

Verfahren B

Zu einer gut gerührten Suspension von 55,3 g (0,4 Mol) wasserfreiem Kaliumcarbonat in 100 ml trockenem Acetonitril tropfte man bei 0 bis 5°C eine Lösung von 30,1 g (0,1 Mol) N-(2,3-Dibrompropionyl)-glycin und 30,4 g (0,22 Mol) Thiobenzoesäure in 100 ml trockenem Acetonitril. Man ließ noch 1 h bei 20°C nachrühren, trug den Ansatz anschließend in 1 l Eiswasser ein und extrahierte zweimal mit je 400 ml Diethylether. Die vereinigten Etherphasen wurden noch einmal mit 500 ml 10 %iger wäßriger Kaliumcarbonatlösung extrahiert und die vereinigten wäßrigen Phasen unter Kühlung mit 2N wäßriger $H_2SO_4$ auf pH 3 gebracht. Man extrahierte zweimal mit je 400 ml Essigsäureethylester, trocknete die organischen Phasen mit wasserfreiem Natriumsulfat und dampfte ein. Nach Säulenchromatographie an desaktiviertem Kieselgel mit dem System Cyclohexan/Essigsäureethylester =

1/1 erhielt man 25,4 g (63 %) N-[2,3-Bis-(benzoylthio)-propionyl]-glycin, Fp. 162 bis 164°C (Essigsäureethylester).

Analog Beispiel 27 wurden erhalten (die Reinigung der Rohprodukte erfolgte durch Säulenchromatographie an desaktiviertem Kieselgel mit den Systemen Cyclohexan/Essigsäureethylester = 3/1 bis 1/1):

28. N-[2,3-Bis-(benzoylthio)-propionyl]-sarcosin, $R_f$ = 0,58 in System I.

29. N-[2,3-Bis-(benzoylthio)-propionyl]-alanin, Fp. 152 bis 155°C (Cyclohexan/Essigsäureethylester), $R_f$ = 0,32 in System II.

30. N-[2,3-Bis-(benzoylthio)-propionyl]-D,L-norleucin als Diastereomerengemisch, Fp. 126 bis 127°C (Diethylether), $R_f$ = 0,39 in System II.

31. N-[2,3-Bis-(benzoylthio)-propionyl]-leucin als Diastereomerengemisch, Fp. 90 bis 93°C (Cyclohexan/Essigsäureethylester), $R_f$ = 0,38 in System II.

32. N-[2,3-Bis-(benzoylthio)-propionyl]-threonin als Diastereomerengemisch, Fp. 156 bis 159°C (Diethylether), $R_f$ = 0,44 und 0,40 in System I.

33. N-[2,3-Bis-(benzoylthio)-propionyl]-cystein als Diastereomerengemisch, Fp. 171 bis 173°C (Essigsäureethylester), $R_f$ = 0,34 in System II.

34. N-[2,3-Bis-(benzoylthio)-propionyl]-L-penicillamin als Diastereomerengemisch, Fp. 114 bis 117°C (Diisopropylether), $R_f$ = 0,43 in System II.

35. N-[2,3-Bis-(benzoylthio)-propionyl]-methionin als Diastereomerengemisch, Fp. 124 bis 127°C (Cyclohexan/ Essigsäureethylester); $R_f$ = 0,38 in System II.

36. N-[2,3-Bis-(benzoylthio)-propionyl]-glutaminsäure-$\gamma$-benzylester als Diastereomerengemisch, Fp. 93 bis 96°C (Diethylether/Petrolether), $R_f$ = 0,36 in System II.

37. $N_\alpha$-[2,3-Bis-(benzoylthio)-propionyl]-glutamin als Diastereomerengemisch, $R_f$ = 0,48 in System I.

38. $N_{\alpha}$-[2,3-Bis-(benzoylthio)-propionyl]-$N_{\varepsilon}$-(benzyloxycarbonyl)-lysin als Diastereomerengemisch, Fp. 107 bis 110°C (Diethylether), $R_f$ = 0,64 in System I.

39. N-[2,3-Bis-(benzoylthio)-propionyl]-D,L-azetidin-2-carbonsäure als Diastereomerengemisch, $R_f$ = 0,41 und 0,37 in System I.

40. N-[2,3-Bis-(benzoylthio)-propionyl]-D,L-piperidin-2-carbonsäure als Diastereomerengemisch, Fp. 162 bis 165°C, $R_f$ = 0,66 in System I.

41. N-[2,3-Bis-(benzoylthio)-propionyl]-D,L-1,2,3,4-tetrahydroisochinolin-3-carbonsäure als Diastereomerengemisch, $R_f$ = 0,67 in System I.

42. N-[2,3-Bis-(benzoylthio)-propionyl]-D,L-α-phenylglycin als Diastereomerengemisch, Fp. 169 bis 179°C (Diethylether), $R_f$ = 0,37 in System II.

43. N-[2,3-Bis-(benzoylthio)-propionyl]-D,L-α-(thien-3-yl)-glycin als Diastereomerengemisch, Fp. 161 bis 164°C (Diethylether), $R_f$ = 0,35 in System II.

44. N-[2,3-Bis-(benzoylthio)-propionyl]-phenylalanin als Diastereomerengemisch, Fp. 104 bis 107°C (Toluol/ Petrolether), $R_f$ = 0,39 in System II.

45. N-[2,3-Bis-(benzoylthio)-propionyl]-tyrosin als Diastereomerengemisch, $R_f$ = 0,27 in System II.

46. N-[2,3-Bis-(benzoylthio)-propionyl]-D,L-(3,4-dimethoxy)-phenylalanin als Diastereomerengemisch, $R_f$ = 0,25 in System II.

47. N-[2,3-Bis-(benzoylthio)-propionyl]-O-benzyl-tyrosin als Diastereomerengemisch, Fp. 173 bis 176°C (Essigsäureethylester/Petrolether), $R_f$ = 0,38 in System II.

48. N-[2,3-Bis-(benzoylthio)-propionyl]-p-chlorphenylalanin als Diastereomerengemisch, $R_f$ = 0,37 in System II.

49. N-[2,3-Bis-(benzoylthio)-propionyl]-D,L-3-(naphth-1-yl)-alanin als Diastereomerengemisch, $R_f$ = 0,37 in System II.

50. N-[2,3-Bis-(benzoylthio)-propionyl]-D,L-3-(naphth-2-yl)-alanin als Diastereomerengemisch, Fp. 140 bis 143°C (Cyclohexan/Essigsäureethylester), $R_f$ = 0,36 in System II.

51. D,L-2-[2,3-Bis-(benzoylthio)-propionamido]-4-phenylbuttersäure als Diastereomerengemisch, Fp. 157 bis 160°C (Essigsäureethylester/Petrolether), $R_f$ = 0,38 in System II.

52. N-[2,3-Bis-(benzoylthio)-propionyl]-tryptophan als Diastereomerengemisch, Fp. 180 bis 183°C (Essigsäureethylester), $R_f$ = 0,34 in System II.

53. N-[2,3-Bis-(benzoylthio)-propionyl]-D-tryptophan als Diastereomerengemisch, Fp. 182 bis 185°C (Essigsäureethylester), $R_f$ = 0,34 in System II.

54. N-[2,3-Bis-(benzoylthio)-propionyl]-N-methyl-tryptophan als Diastereomerengemisch, $R_f$ = 0,35 in System II.

55. N-[2,3-Bis-(benzoylthio)-propionyl]-prolyl-prolin als Diastereomerengemisch, $R_f$ = 0,22 in System I.

56. N-[2,3-Bis-(benzoylthio)-propionyl]-phenylalanyl-alanin als Diastereomerengemisch, Fp. 110 bis 113°C (Diethylether), $R_f$ = 0,62 in System I.

57. N-[2,3-Bis-(benzoylthio)-propionyl]-tryptophyl-prolin als Diastereomerengemisch, $R_f$ = 0,62 in System I.

Beispiel 58

N-[2,3-Bis-(benzoylthio)-propionyl]-serin

Man verfuhr wie in Beispiel 27 und verwendete Serin statt Glycin. Nach Säulenchromatographie an desaktiviertem Kieselgel mit dem System Cyclohexan/Essigsäureethylester = 1/1 erhielt man N-[2,3-Bis-(benzoylthio)--propionyl]-serin als Diastereomerengemisch. Fraktionierte Kristallisation aus Essigsäureethylester lieferte die Diastereomeren N-[(R)-2,3-Bis--(benzoylthio)-propionyl]-serin, Fp. 184 bis 187°C, $R_f$ = 0,35 in System I, $[\alpha]_D^{20}$= -60° (c = 1 in Chloroform/Methanol = 9/1) und N-[(S)--2,3-Bis-(benzoylthio)-propionyl]-serin, Fp. 147 bis 150°C, $R_f$ = 0,39 in System I, $[\alpha]_D^{20}$= +116° (c = 1 in Chloroform/ Methanol = 9/1).

Beispiel 59

N-[2,3-Bis-(benzoylthio)-propionyl]-valin

Man verfuhr wie in Beispiel 27 und verwendete Valin statt Glycin. Nach Säulenchromatographie an desaktiviertem Kieselgel mit dem System Cyclohexan/Essigsäureethylester = 3/1 erhielt man N-[2,3-Bis-(benzoylthio)-propionyl]-valin als Diastereomerengemisch. Fraktionierte Kristallisation aus Diethylether und Diethylether/Petrolether liefert die Diastereomeren N-[(S)-2,3-Bis-(benzoylthio)-propionyl]-valin, Fp. 157 bis 160°C, $R_f$ = 0,39 in System II, $[\alpha]_D^{20}$= +152° (c = 1 in Chloroform), und N-[(R)-2,3-Bis-(benzoylthio)-propionyl]-valin, Fp. 83 bis 90°C, $R_f$ = 0,37 in System II, $[\alpha]_D^{20}$= -69° (c = 1 in Chloroform).

Beispiel 60

N-[2,3-Bis-(benzoylthio)-propionyl]-isoleucin

Man verfuhr wie in Beispiel 27 und verwendete Isoleucin statt Glycin. Nach Säulenchromatographie an desaktiviertem Kieselgel mit dem System Cyclohexan/Essigsäureethylester = 3/1 erhielt man N-[2,3-Bis-(benzoylthio)-propionyl]-isoleucin als Diastereomerengemisch. Fraktionierte Kristallisation aus Diethylether und Diisopropylether lieferte die Diastereomeren N-[(S)-2,3-Bis-(benzoylthio)-propionyl]-isoleucin, Fp. 151 bis 153°C , $R_f$ = 0,39 in System II, $[\alpha]_D^{20}$= +135° (c = 1 in Chloroform), und N-[(R)-2,3-Bis-(benzoylthio)-propionyl]-isoleucin, Fp. 94 bis 95°C, $R_f$ = 0,39 in System II, $[\alpha]_D^{20}$= -65° (c = 1 in Chloroform).

Beispiel 61

N-[2,3-Bis-(benzoylthio)-propionyl]-L-thiazolidin-4-carbonsäure

Man verfuhr wie in Beispiel 27 und verwendete L-Thiazolidin-4-carbonsäure statt Glycin. Nach Säulenchromatographie an desaktiviertem Kieselgel mit dem System Cyclohexan/Essigsäureethylester = 2/1 erhielt man die beiden Diastereomeren N-[(S)-2,3-Bis-(benzoylthio)-propionyl]-L-thiazolidin-4-carbonsäure, Fp. 133 bis 138°C (Diethylether), $R_f$ = 0,61 in System I, $[\alpha]_D^{20}$= +82° (c = 1 in Chloroform), und N-[(R)-2,3-Bis-(benzoylthio)-propionyl]-L-thiazolidin-4-carbonsäure, $R_f$ = 0,57 in System I, $[\alpha]_D^{20}$= -145° (c = 1 in Chloroform).

Analog wurden erhalten:

62. N-[(R)-2,3-Bis-(benzoylthio)-propionyl]-prolylphenylalanin, $R_f$ = 0,50 in System I, $[\alpha]_D^{20}$= -133° (c = 1 in Chloroform)

63. N-[(S)-2,3-Bis-(benzoylthio)-propionyl]-prolyl-phenylalanin, $R_f$ = 0,45 in System I, $[\alpha]_D^{20}$ = +41° (c = 1 in Chloroform)

Beispiel 64

N-[2,3-Bis-(benzoylthio)-propionyl]-alanyl-prolin

Man verfuhr wie in Beispiel 27 und verwendete Alanyl-prolin statt Glycin. Das Umsetzungsprodukt kristallisierte direkt aus dem Reaktionsansatz. Umkristallisation aus Isopropanol lieferte N-[2,3-Bis-(benzoylthio)--propionyl]-alanyl-prolin als Diastereomerengemisch, Fp. 185 bis 186°C, $R_f$ = 0,40 in System I, $[\alpha]_D^{20}$ = -39° (c = 1 in Chloroform). Fraktionierte Kristallisation des Gemisches aus Essigsäureethylester und Essigsäureethylester/Diethylether lieferte die Diastereomeren: N-(R)-2,3-Bis--(benzoylthio)-propionyl]-alanyl-prolin, Fp. 196 bis 197°C, $R_f$ = 0,40 in System I, $[\alpha]_D^{20}$ = -122° (c = 1 in Chloroform), und N-[(S)-2,3-Bis--(benzoylthio)-propionyl]-alanylprolin, Fp. 172 bis 173°C, $R_f$ = 0,40 in System I, $[\alpha]_D^{20}$ = +36° (c = 1 in Chloroform).

Beispiel 65

N-[(S)-2,3-Bis-(benzoylthio)-propionyl]-prolin,

4,4 g (25 mMol) Prolin-tert.-butylester (Rohprodukt, erhalten durch Hydrierung von 7,6 g Z-Prolin-tert.-butylester) in 100 ml Tetrahydrofuran wurden unter Kühlung auf 0°C mit einer Lösung von 8,7 g (25 mMol) (S)-2,3--Bis-(benzoylthio)-propionsäure und 4,8 g (25 mMol) 1-Ethyl-3-(3-dimethylaminopropyl)-carbodiimid-hydrochlorid in 125 ml Tetrahydrofuran/Wasser = 4/1 versetzt. Nach 1 h Rühren bei 0°C wurde noch 1 h bei 20°C nachgerührt, der Ansatz zwischen Essigsäureethylester und Wasser ausgeschüttelt und die organische Phase nach Trocknen mit wasserfreiem Natriumsulfat unter vermindertem Druck eingedampft. Säulenchromatographie an desaktiviertem Kieselgel mit dem System Cyclohexan/Essigsäureethylester = 2/1 lieferte 9,0 g (72 %) N-[(S)-2,3-Bis-(benzoylthio)-propionyl]-prolin--tert.-butylester. Das Rohprodukt wurde in 50 ml trockenem Dioxan gelöst und mit 50 ml 6N HCl/Dioxan 1 h bei 20°C stehen gelassen. Nach Ausschüttelung zwischen Essigsäureethylester und Wasser, zweimaligem Waschen der organischen Phase mit Wasser, Trocknen mit wasserfreiem Natriumsulfat und Eindampfen unter vermindertem Druck erhielt man 7,8 g (70 %) N-[(S)--2,3-Bis-(benzoylthio)-propionyl]-prolin, Fp. 155 bis 158°C (Cyclohexan/Essigsäureethylester), $R_f$ = 0,56 in System I, $[\alpha]_D^{20}$ = +76° (c = 1 in Chloroform).

Analog Beispiel 65 wurden erhalten (die Reinigung der Rohprodukte erfolgte durch Säulenchromatographie an desaktiviertem Kieselgel mit den Systemen Cyclohexan/Essigsäureethylester = 3:1 bis 1:1).

66. N-[(R)-2,3-Bis-(benzoylthio)-propionyl]-prolin, $R_f$ = 0,54 in System I, $[\alpha]_D^{20}$ = -117° (c = 1 in Chloroform).

67. N-[(S)-2,3-Bis-(benzoylthio)-propionyl]-tryptophan, $R_f$ = 0,34 in System II, $[\alpha]_D^{20}$ = +84° (c = 1 in Chloroform).

68. N-[(R)-2,3-Bis-(benzoylthio)-propionyl]-tryptophan, $R_f$ = 0,34 in System II, $[\alpha]_D^{20}$ = -77° (c = 1 in Chloroform).

69. N-[(S)-2,3-Bis-(benzoylthio)-propionyl]-alanyl-prolin, Fp. 175 bis 176°C (Essigsäureethylester), $R_f$ = 0,40 in System I, $[\alpha]_D^{20}$ = +36° (c = 1 in Chloroform).

70. N-[(R)-2,3-Bis-(benzoylthio)-propionyl]-alanyl-prolin, Fp. 195 bis 196°C (Essigsäureethylester), $R_f$ = 0,40 in System I, $[\alpha]_D^{20}$ = -125° (c = 1 in Chloroform).

Beispiel 71

N-[(S)-2,3-Bis-(benzoylthio)-propionyl]-valyl-prolin,

Man verfuhr wie in Beispiel 61 und verwendete N-[(S)-2,3-Bis-(benzoylthio)-propionyl]-valin (Beispiel 59) statt (S)-2,3-Bis-(benzoylthio)-propionsäure. Nach Säulenchromatographie an desaktiviertem Kieselgel mit dem System Cyclohexan/Essigsäureethylester = 1/1 erhielt man N-[(S)-2,3-Bis-(benzoylthio)-propionyl]-valyl-prolin, Fp. 85 bis 93°C (Diethylether/Pentan), $R_f$ = 0,54 in System I, $[\alpha]_D^{20}$ = +53° (c = 1 in Chloroform).

Analog Beispiel 71 wurden hergestellt:

72. N-[(R)-2,3-Bis-(benzoylthio)-propionyl]-valyl-prolin, Fp. 97 bis 100°C (Diethylether), $R_f$ = 0,54 in System I, $[\alpha]_D^{20}$ = -114° (c = 1 in Chloroform).

73. N-[(S)-2,3-Bis-(benzoylthio)-propionyl]-valyl-tryptophan, Fp. 173 bis 181°C (Toluol), $R_f$ = 0,60 in System I.

74. N-[(R)-2,3-Bis-(benzoylthio)-propionyl]-valyl-tryptophan, Fp. 205 bis 208°C (Toluol), $R_f$ = 0,60 in System I.

Beispiel 75

N-[3,3'-Bis-(benzoylthio)-isobutyryl]-glycin

a) Herstellung des Ausgangsmaterials

Zu einer Lösung von 10,6 g (50 mMol) 2-Iodmethyl-acrylsäure und 13,8 g (100 mMol) Kaliumcarbonat in 100 ml Dioxan/Wasser = 2/3 wurden unter Rühren 14,5 g (105 mMol) Thiobenzoesäure zugetropft. Nach 3 h Rühren bei Raumtemperatur wurde der Ansatz mit 500 ml Wasser verdünnt, mit 1N wäßriger Schwefelsäure auf pH 3 gebracht und zweimal mit je 300 ml Essigsäureethylester ausgeschüttelt. Die vereinigten organischen Phasen wurden noch einmal mit wenig Wasser gewaschen, mit wasserfreiem Natriumsulfat getrocknet und unter vermindertem Druck eingedampft. Nach Kristallisation des Rückstandes aus Cyclohexan erhielt man 15,5 g (86 %) 3,3'-Bis--(benzoylthio)-isobuttersäure, Fp. 107 bis 109°C, Rf = 0,45 in System II.

b) Herstellung des Endprodukts

14,4 g (40 mMol) 3,3'-Bis-(benzoylthio)-isobuttersäure wurden in 120 ml Toluol suspendiert und unter Kühlung mit 25,4 g (200 mMol) Oxalsäuredichlorid versetzt. Man erwärmte 2 h auf 50°C, dampfte anschließend unter vermindertem Druck ein und entfernte überschüssiges Oxalsäuredichlorid durch mehrmaliges Koevaporieren mit Toluol.

Der Rückstand wurde in 150 ml trockenem Dioxan aufgenommen und zusammen mit 3,0 g (40 mMol) Glycin 2 h unter Rückfluß gekocht. Anschließend dampfte man unter vermindertem Druck ein. Säulenchromatographie an desaktiviertem Kieselgel mit dem System Cyclohexan/Essigsäureethylester = 2/1 lieferte 12,7 g (76 %) N-[3,3'-Bis-(benzoylthio)-isobutyryl]--glycin, Fp. 113 bis 114°C (Diethylether), $R_f$ = 0,58 in System I.

Analog erhielt man (die Reinigung der Rohprodukte erfolgte durch Säulenchromatographie an desaktiviertem Kieselgel mit den Systemen Cyclohexan/Essigsäureethylester = 3/1 bis 1/1):

76. N-[3,3'-Bis-(benzoylthio)-isobutyryl]-sarcosin, Fp. 130 bis 132°C (Diethylether/Petrolether), $R_f$ = 0,59 in System I.

77. N-[3,3'-Bis-(benzoylthio)-isobutyryl]-alanin, Fp. 145 bis 146°C (Diethylether), $R_f$ = 0,31 in System II.

78. N-[3,3'-Bis-(benzoylthio)-isobutyryl]-D-alanin, Fp. 144 bis 145°C (Diethylether), $R_f$ = 0,31 in System II.

79. N-[3,3'-Bis-(benzoylthio)-isobutyryl]-D,L-norleucin, Fp. 132 bis 133°C (Diethylether/Petrolether), $R_f$ = 0,39 in System II.

80. N-[3,3'-Bis-(benzoylthio)-isobutyryl]-valin, $R_f$ = 0,38 in System II.

81. N-[3,3'-Bis-(benzoylthio)-isobutyryl]-isoleucin, $R_f$ = 0,38 in System II.

82. N-[3,3'-Bis-(benzoylthio)-isobutyryl]-leucin, Fp. 93 bis 94°C (Diethylether/Petrolether), $R_f$ = 0,37 in System II.

83. N-[3,3'-Bis-(benzoylthio)-isobutyryl]-methionin, Fp. 113 bis 114°C (Diethylether/Petrolether), $R_f$ = 0,36 in System II.

84. N-[3,3'-Bis-(benzoylthio)-isobutyryl]-serin, $R_f$ = 0,38 in System I.

85. N-[3,3'-Bis-(benzoylthio)-isobutyryl]-cystein, $R_f$ = 0,35 in System II.

86. N-[3,3'-Bis-(benzoylthio)-isobutyryl]-prolin, Fp. 137 bis 138°C (Essigsäureethylester), $R_f$ = 0,56 in System I.

87. N-[3,3'-Bis-(benzoylthio)-isobutyryl]-D-prolin, Fp. 136 bis 138°C (Essigsäureethylester), $R_f$ = 0,56 in System I.

88. N-[3,3'-Bis-(benzoylthio)-isobutyryl]-thiazolidin-4-carbonsäure, Fp. 127 bis 129°C (Diethylether), $R_f$ = 0,60 in System I.

89. N-[3,3'-Bis-(benzoylthio)-isobutyryl]-D,L-azetidin-2-carbonsäure, $R_f$ = 0,43 in System I.

90. N-[3,3'-Bis-(benzoylthio)-isobutyryl]-D,L-piperidin-2- carbonsäure, $R_f$ = 0,67 in System I.

91. N-[3,3'-Bis-(benzoylthio)-isobutyryl]-D,L-1,2,3,4-tetrahydroisochinolin-3-carbonsäure, $R_f$ = 0,66 in System I.

92. N-[3,3'-Bis-(benzoylthio)-isobutyryl]-D,L- -phenyl-glycin, Fp. 158 bis 160°C (Diethylether/Petrolether), $R_f$ = 0,37 in System II.

93. N-[3,3'-Bis-(benzoylthio)-isobutyryl]-phenylalanin, Fp. 57 bis 60°C (Diethylether/Petrolether), $R_f$ = 0,39 in System II.

94. N-[3,3'-Bis-(benzoylthio)-isobutyryl]-D-phenylalanin, Fp. 59 bis 61°C (Diethylether/Petrolether), $R_f$ = 0,39 in System II.

95. N-[3,3'-Bis-(benzoylthio)-isobutyryl]-tyrosin, Fp. 71 bis 73°C (Petrolether), $R_f$ = 0,27 in System II.

96. N-[3,3'-Bis-(benzoylthio)-isobutyryl]-D,L-(3,4-dimethoxy)-phenylalanin, Fp. 159 bis 161°C (Essigsäureethylester), $R_f$ = 0,25 in System II.

97. N-[3,3'-Bis-(benzoylthio)-isobutyryl]-O-benzyl-tyrosin, $R_f$ = 0,36 in System II.

98. N-[3,3'-Bis-(benzoylthio)-isobutyryl]-p-chlorphenylalanin, Fp. 143 bis 146°C (Diethylether), $R_f$ = 0,35 in System II.

99. N-[3,3'-Bis-(benzoylthio)-isobutyryl]-D,L-3-(naphth-1-yl)-alanin, $R_f$ = 0,37 in System II.

100. N-[3,3'-Bis-(benzoylthio)-isobutyryl]-D,L-3-(naphth-2-yl)-alanin, $R_f$ = 0,37 in System II.

101. D,L-2-[3,3'-Bis-(benzoylthio)-isobutyramido]-4-phenylbuttersäure, Fp. 135 bis 136°C (Diethylether/Cyclohexan), $R_f$ = 0,38 in System II.

102. N-[3,3'-Bis-(benzoylthio)-isobutyryl]-tryptophan, Fp. 135 bis 139°C (Diethylether/Petrolether), $R_f$ = 0,35 in System II.

103. N-[3,3'-Bis-(benzoylthio)-isobutyryl]-D-tryptophan, Fp. 137 bis 140°C (Diethylether/Petrolether), $R_f$ = 0,35 in System II.

104. N-[3,3'-Bis-(benzoylthio)-isobutyryl]-N-methyl-tryptophan, $R_f$ = 0,37 in System II.

105. N-[3,3'-Bis-(benzoylthio)-isobutyryl]-alanyl-prolin, Fp. ab 60°C (Methylenchlorid/Petrolether), $R_f$ = 0,39 in System I.

106. N-[3,3'-Bis-(benzoylthio)-isobutyryl]-prolyl-phenylalanin, Fp. 134-135°C(Diethylether), $R_f$ = 0,47 in System I.

107. N-[3,3'-Bis-(benzoylthio)-isobutyryl]-prolyl-prolin, $R_f$ = 0,23 in System I.

108. N-[3,3'-Bis-(benzoylthio)-isobutyryl]-phenylalanyl-alanin, $R_f$ = 0,62 in System I.

109. N-[3,3'-Bis-(benzoylthio)-isobutyryl]-valyl-prolin, $R_f$ = 0,54 in System I.

110. N-[3,3'-Bis-(benzoylthio)-isobutyryl]-valyl-tryptophan, $R_f$ = 0,58 in System I.

Beispiel A

Auf einer Tablettenpresse werden in üblicher Weise Tabletten folgender Zusammensetzung gepreßt:

40 mg Substanz des Beispiels 62
120 mg Maisstärke
13,50 mg Gelatine
45 mg Milchzucker
22,5 mg Talk
2,25 mg Aerosil$^R$ (chemisch reine Kieselsäure in submikroskopisch feiner Verteilung)
6,75 mg Kartoffelstärke (als 6 %iger Kleister)

Beispiel B

In üblicher Weise werden Dragees folgender Zusammensetzung hergestellt:

20 mg Substanz des Beispiels 62
60 mg Kernmasse
60 mg Verzuckerungsmasse

Die Kernmasse besteht aus 9 Teilen Maisstärke, 3 Teilen Milchzucker und 1 Teil Luviskol$^R$ VA 64 (Vinylpyrrolidon-Vinylacetat-Mischpolymerisat 60:40, vgl. Pharm. Ind. 1962, 586). Die Verzuckerungsmasse besteht aus 5 Teilen Rohrzucker, 2 Teilen Maisstärke, 2 Teilen Calciumcarbonat und 1 Teil Kalk.

Patentansprüche

1. Benzoylthioverbindungen der Formel I

$$\begin{array}{l} \qquad\qquad C_6H_5-CO-X \\ \qquad\qquad\qquad\quad (CH_2)_q \\ C_6H_5-CO-S-(CH_2)_n-CH-CO-NR^1-CHR^2-CO-(NR^3-CHR^4-CO)_m-OH \end{array} \qquad I,$$

worin

$R^1$ und $R^3$ gleich oder verschieden sind und Wasserstoffatome oder $C_{1-4}$-Alkylgruppen darstellen,

$R^2$ und $R^4$ gleich oder verschieden sind und Wasserstoff, einen gegebenenfalls durch eine Hydroxy-, Mercapto-, $C_{1-4}$-Alkylthio-, Carbonsäureester-, Carbonamido- oder Acylaminogruppe substituierten $C_{1-6}$-Alkylrest oder einen gegebenenfalls im Arylrest durch Hydroxy-, $C_{1-4}$-Alkoxy-, Arylalkoxygruppen oder Halogenatome substituierten Aryl-, Heteroaryl-, Arylalkylen- oder Heteroarylalkylenrest bedeuten, wobei $R^1$ und $R^2$ und/oder $R^3$ und $R^4$ zusammen auch die Reste $-(CH_2)_p-$ ($p = 2, 3, 4$), $-CH_2-S-CH_2-$ oder $-CH_2-C_6H_4-CH_2-$(ortho) sein können,

X eine NH-Gruppe oder ein Schwefelatom ist,

m und n gleich oder verschieden sind und die Zahlen 0 oder 1 bedeuten und

q - falls X = S - die Zahl 0 oder 1 oder - falls X = NH - die Zahl 0 darstellt,

sowie deren Salze mit physiologisch verträglichen Basen.

2. Benzoylthioverbindungen der Formel I gemäß Anspruch 1, dadurch gekennzeichnet, daß

$R^1$ und $R^3$ Wasserstoffatome darstellen,

$R^2$ Wasserstoff, einen $C_1$-$C_4$-Alkyl-, Benzyl-, Naphthylmethylen- oder Indol-3-yl-methylenrest bedeutet,

$R^4$ ein $C_{1-4}$-Alkyl-, Benzyl- oder Indol-3-ylmethylenrest ist wobei $R^1$ und $R^2$ und/oder $R^3$ und $R^4$ auch zusammen den $-(CH_2)_3-$ oder $-CH_2-S-CH_2-$Rest darstellen können, sowie

X ein Schwefelatom ist und

n, m und q gleich oder verschieden sind und die Zahlen 0 oder 1 bedeuten,

sowie deren Salze mit physiologisch verträglichen Säuren.

3. Verfahren zur Herstellung der Verbindungen der Formel I gemäß Anspruch 1, <u>dadurch gekennzeichnet</u>, daß man

a) eine Säure der Formel II

$$\begin{array}{l} \qquad\quad C_6H_5-CO-X \\ \qquad\qquad\qquad\quad | \\ \qquad\qquad\quad\ \ (CH_2)_q \\ \qquad\qquad\qquad\quad | \\ C_6H_5-CO-S-(CH_2)_n-CH-COOH \end{array} \qquad II,$$

worin X, n und q die angegebene Bedeutung besitzen, mit einer Verbindung der Formel III

$$R^1NH-CHR^2-CO-(NR^3-CHR^4-CO)_m-OH \qquad III,$$

worin $R^1$, $R^2$, $R^3$, $R^4$ und m die angegebene Bedeutung besitzen kondensiert oder

b) - falls X ein S-Atom ist - eine Verbindung der Formel

$$\begin{array}{l} \qquad\qquad Y-(CH_2)_q \\ \qquad\qquad\qquad\quad | \\ Y-(CH_2)_n-CH-CO-NR^1-CHR^2-CO-(NR^3-CHR^4-CO)_m-OH \end{array} \qquad (IV),$$

worin $R^1$, $R^2$, $R^3$, $R^4$, m, n und q die angegebene Bedeutung besitzen und Y ein Halogenatom ist,

mit Thiobenzoesäure umsetzt

und die so erhaltenen Verbindungen gegebenenfalls in ihre Salze mit physiologisch verträglichen Basen überführt.

4. Arzneimittel, enthaltend eine Verbindung gemäß Anspruch 1.

5. Benzoylthioverbindung gemäß Anspruch 1 zur Verwendung bei der Bekämpfung von Krankheiten.